# FASCICULE DE BREVET EUROPEEN

(11) **EP 0 688 575 B1**
(45) Date de publication et mention de la délivrance du brevet: **22.09.1999**
(21) Numéro de dépôt: 95401498.1
(22) Date de dépôt: 23.06.1995
(51) Int. Cl.: A61M 27/00, F16K 31/08

(54) **Valve sous-cutanée et son dispositif de réglage externe**
Subkutanesventil und seine externe Einstellungsvorrichtung
Valve for subcutaneous use and its external control device

(30) Priorité: 24.06.1994 FR 9407790
(43) Date de publication de la demande: 27.12.1995
(73) Titulaire: SOPHYSA Société Anonyme, F-91893 Orsay (FR)
(72) Inventeur: Negre, Philippe, F-91440 Bures-sur-Yvette (FR)
(74) Mandataire: Leszczynski, André

(56) Documents cités:
- EP-A- 0 060 369
- WO-A-91/08001
- FR-A- 2 354 103
- US-A- 4 551 128
- US-A- 4 676 772

## Description

La présente invention concerne une valve sous-cutanée destinée à des applications thérapeutiques et son dispositif de réglage externe qui permet de modifier à travers des tissus cutanés un passage ou une distribution de liquide dans des prothèses ou des systèmes implantés.

Parmi les applications thérapeutiques de la valve selon la présente invention, on peut citer le traitement de l'hydrocéphalie qui consiste à dériver le liquide céphalorachidien contenu dans les ventricules de la cavité crânienne vers tout autre site de résorption.

Une valve connue pour cette application est décrite dans le brevet français de la Société Déposante n° 81 05389.

Une telle valve comporte un corps constitué d'une chambre interne sensiblement cylindrique et plate, un conduit d'entrée et un conduit d'évacuation ménagés dans la paroi latérale de ladite chambre et aptes à être respectivement connectés à un cathéter d'amenée et un cathéter de drainage de liquide, un rotor en forme de barreau apte à tourner dans ladite chambre autour de son axe central, un clapet anti-retour constitué d'une bille et d'un siège en forme de cône au niveau de l'extrémité interne du conduit d'entrée, un ressort à lame semi-circulaire, fixé audit rotor, parallèle à la paroi latérale de la chambre et comprimant la bille dans son siège de manière à réguler et le cas échéant bloquer le passage de liquide dans la chambre par le conduit d'entrée, deux micro-aimants fixes montés dans le rotor et disposés de part et d'autre de l'axe central de la chambre, et des moyens de verrouillage du rotor dans une position déterminée, les moyens de verrouillage comportant un ergot en saillie d'une extrémité du rotor et des cavités de réception des ergots ménagées circulairement dans la paroi latérale du corps de valve, lesdites cavités ayant une forme adaptée pour retenir lesdits ergots.

Dans la mesure où le rotor comporte des micro-aimants, il est possible au moyen d'un aimant que l'on place à la verticale de la valve, d'entraîner en rotation le rotor à travers la paroi de la valve et les tissus cutanés qui la recouvrent lorsque la valve est implantée. On peut ainsi régler à distance et de l'extérieur le rotor dans ses différentes positions de verrouillage, et par conséquent modifier la pression et le débit de fonctionnement de la valve.

Cependant, ce type de valve à rotor magnétique présente l'inconvénient de pouvoir être déréglé sous l'action d'un champ magnétique ou électro-magnétique externe de forte puissance, tel que celui rencontré en imagerie à résonance magnétique nucléaire.

Cet inconvénient est très inconfortable pour les porteurs de valves du type précité qui doivent faire rerégler leur valve après chaque examen utilisant des techniques comme l'imagerie à résonance magnétique nucléaire. Les documents US-A-4 551 128 et US-A-4 676 772 montrent également des valves, qui de plus, sont actionnées par un aimant.

La présente invention a pour but de fournir une valve sous-cutanée qui ne peut pas être déréglée par un champ magnétique externe autre que celui provenant d'un dispositif de réglage magnétique particulier.

La présente invention a pour objet une valve sous-cutanée destinée à des applications thérapeutiques permettant le réglage externe d'une distribution de liquide, ladite valve comportant un corps constitué d'une chambre interne sensiblement cylindrique et plate, un conduit d'entrée et un conduit d'évacuation ménagés dans la paroi latérale de ladite chambre et aptes à être respectivement connectés à un cathéter d'amenée et un cathéter de drainage de liquide, un rotor apte à tourner dans ladite chambre autour de son axe central, un clapet anti-retour, tel une bille, formé au niveau de l'extrémité interne du conduit d'entrée, un ressort à lame courbe, de préférence semi-circulaire, fixé audit rotor, parallèle à la paroi latérale de la chambre et comprimant le clapet dans son siège de manière à réguler, et le cas échéant bloquer le passage de liquide dans la chambre par le conduit d'entrée, deux micro-aimants montés dans le rotor et disposés de part et d'autre de l'axe central de la chambre et des moyens de verrouillage du rotor dans une position déterminée, caractérisée par le fait que les micro-aimants du rotor sont mobiles linéairement dans ledit rotor selon une direction sensiblement radiale de celui-ci, de manière à actionner lesdits moyens de verrouillage.

Le rotor est avantageusement constitué d'un barreau en forme de H dont les branches latérales servent de moyens de guidage pour les micro-aimants mobiles.

Les moyens de verrouillage comportent de préférence un ergot en saillie de pièces mobiles logeant chacune un micro-aimant et des cavités de réception des ergots ménagées circulairement dans la chambre, lesdites cavités ayant une forme adaptée pour retenir lesdits ergots.

Dans un premier mode de réalisation de la valve selon la présente invention, les micro-aimants mobiles sont disposés de manière à s'attirer, leurs faces en regard étant de polarités opposées et les cavités sont ménagées sur la périphérie d'une partie centrale de la paroi supérieure ou inférieure du corps de valve, les ergots faisant saillie des pièces mobiles respectives perpendiculairement à ladite paroi supérieure ou inférieure.

La paroi supérieure ou inférieure du corps de valve est avantageusement constituée d'un couvercle amovible qui comporte ladite partie centrale.

Dans un second mode de réalisation de la valve selon la présente invention, les micro-aimants mobiles sont de même polarité et lesdites cavités sont ménagées sur la périphérie de la paroi latérale de la chambre, les ergots faisant radialement saillie des pièces mobiles respectives vers ladite paroi latérale.

La présente invention a également pour objet un dispositif de réglage externe de la valve selon la présente invention.

Selon un premier mode de réalisation, le dispositif de réglage externe selon la présente invention, est constitué de deux aimants dont les faces en regard sont de polarités opposées et de masse magnétique supérieure à celles des micro-aimants mobiles, lesdits aimants étant montés sur un support commun, de préférence annulaire ou en forme de fer à cheval, et espacés d'une distance sensiblement égale ou supérieure à celle séparant les pôles extérieurs des deux micro-aimants mobiles lorsque ces derniers actionnent les moyens de verrouillage.

Selon un second mode de réalisation, le dispositif de réglage externe selon la présente invention, est constitué d'une succession circulaire de pôles aimantés, montés sur un support, de préférence annulaire, les pôles de même polarité étant disposés diamétralement deux par deux et espacés d'une distance sensiblement égale ou supérieure à celle séparant les pôles extérieurs des deux micro-aimants mobiles lorsque ces derniers actionnent les moyens de verrouillage.

Selon un troisième mode de réalisation, le dispositif de réglage externe selon la présente invention, comporte un support, de préférence annulaire, et des moyens électromagnétiques montés circulairement sur ledit support, et disposés radialement en direction des cavités de réception des ergots, lesdits moyens électromagnétiques étant aptes à envoyer des séquences d'impulsions électromagnétiques codées correspondant aux différentes positions de verrouillage du rotor dans la chambre.

Pour mieux faire comprendre l'objet de la présente invention, on va en décrire ci-après, à titre d'exemple purement illustratif et non limitatif, plusieurs modes de réalisation représentés sur le dessin annexé, dans lequel :
- la figure 1 est une vue de dessus, partiellement en coupe, de la valve selon un premier mode de réalisation de l'invention,
- la figure 2 est une vue en coupe suivant la ligne II-II de la valve de la figure 1,
- la figure 3 est une vue de dessus d'un premier mode de réalisation du dispositif de réglage externe selon la présente invention placé à la verticale de la valve de la figure 1,
- la figure 4 est une vue de dessus, partiellement en coupe, de la valve selon un second mode de réalisation de l'invention,
- la figure 5 est une vue en coupe, suivant la ligne V-V, de la valve de la figure 4,
- la figure 6 est une vue latérale de la valve de la figure 4,
- la figure 7 est une vue de dessus d'un second mode de réalisation du dispositif de réglage externe selon la présente invention,
- la figure 8 est une vue de dessus d'un troisième mode de réalisation du dispositif de réglage externe selon la présente invention, placé à la verticale de la valve de la figure 4.

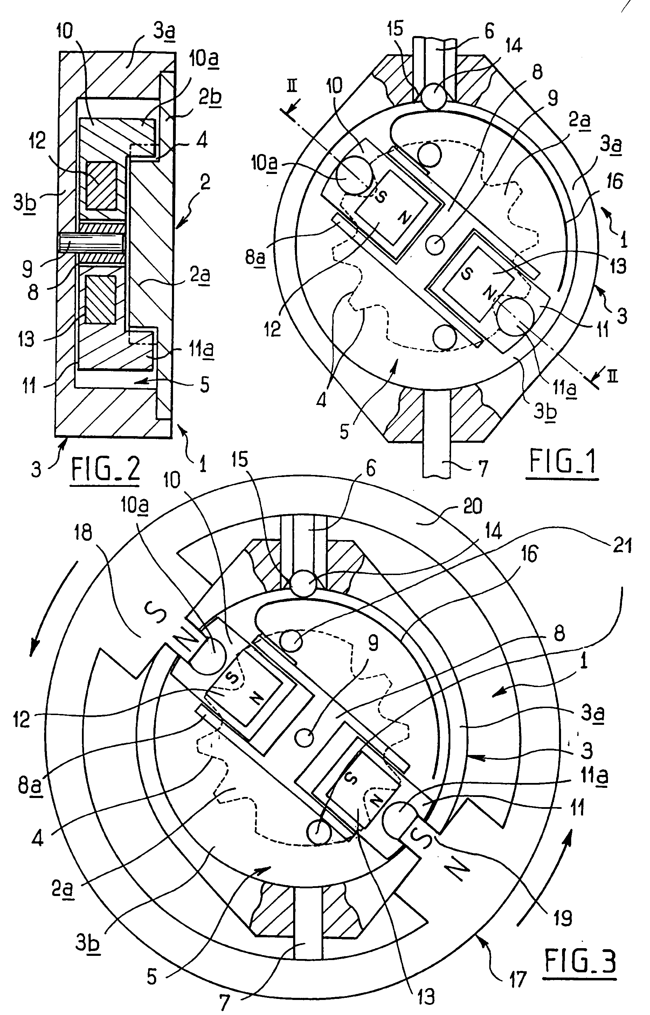

On a représenté sur la figure 1 une vue de dessus d'un corps de valve 1, privé de son couvercle supérieur 2, pour plus de clarté.

Le corps de valve 1 est constitué d'un couvercle supérieur 2 et d'un boîtier 3.

Le couvercle supérieur 2 représenté en coupe sur la figure 2, comporte une partie centrale sensiblement cylindrique 2a séparée par un épaulement d'une partie plane 2b en forme de collerette.

Des cavités 4, représentées en trait interrompu sur les figures 1 et 3, sont ménagées sur la périphérie de la partie centrale 2a.

La partie périphérique de la collerette 2b prend appui dans un évidement annulaire ménagé dans l'extrémité supérieure de la paroi latérale 3a du boîtier 3.

Le corps de valve 1 comporte une chambre interne 5 cylindrique et plate ménagée entre la paroi inférieure 3b, la paroi latérale 3a du boîtier 3 et le couvercle supérieur 2.

Un conduit d'entrée 6 et un conduit d'évacuation 7 sont ménagés dans la paroi latérale 3a du boîtier 3, les extrémités internes desdits conduits 6 et 7 étant disposées diamétralement dans la chambre 5.

Le conduit d'entrée 6 et le conduit d'évacuation 7 sont aptes à être respectivement connectés à un cathéter d'amenée et un cathéter de drainage de liquide, non représentés sur le dessin.

Un rotor 8 constitué d'un barreau en forme de H, est monté tournant dans la chambre 5 autour de son axe central 9. Des éléments de butée 21 en saillie de la paroi inférieure 3b du boîtier 3 sont prévus pour limiter le déplacement en rotation du rotor 8.

Le rotor 8 peut avantageusement être réalisé en matière plastique.

Le rotor 8 comporte des branches latérales 8a qui servent de moyens de guidage, de part et d'autre de l'axe central 9, à des pièces mobiles 10 et 11 logeant chacune un micro-aimant 12 et 13, les faces en regard des micro-aimants étant de polarités opposées (N et S).

Les pièces mobiles 10 et 11 peuvent se déplacer linéairement dans le rotor 8 selon une direction sensiblement radiale de celui-ci de manière à actionner des moyens de verrouillage.

Les moyens de verrouillage sont constitués d'ergots cylindriques 10a et 11a respectivement en saillie des pièces mobiles 10 et 11 et de la succession circulaire de cavités 4 aptes à recevoir lesdits ergots 10a et 11a.

Le corps de valve 1 comporte un clapet anti-retour constitué d'une bille 14 et d'un siège 15 en forme de cône, disposé au niveau de l'extrémité interne du conduit d'entrée 6.

Un ressort à lame semi-circulaire 16 est fixé à une branche latérale 8a du rotor 8, est parallèle à la paroi latérale 3a de la chambre 5 et comprime la bille 14 dans son siège 15 de manière à réguler, et le cas échéant bloquer le passage du liquide dans la chambre 5 par le conduit d'entrée 6.

Sur la figure 3, on a représenté un dispositif de réglage externe 17 particulièrement adapté au premier mode de réalisation du corps de valve 1.

Le dispositif de réglage externe 17 est constitué de deux aimants 18 et 19, par exemple en samarium-cobalt dont les faces en regard sont de polarités opposées (N et S) et de masse magnétique supérieure à celle des micro-aimants mobiles 12 et 13.

Les aimants 18 et 19 sont montés sur un support annulaire commun 20, par exemple en fer doux, diamètralement opposés et espacés d'une distance sensiblement égale ou supérieure à la distance séparant les pôles extérieurs des deux micro-aimants mobiles 12 et 13 lorsque les ergots 10a et 11a sont engagés dans les cavités 4.

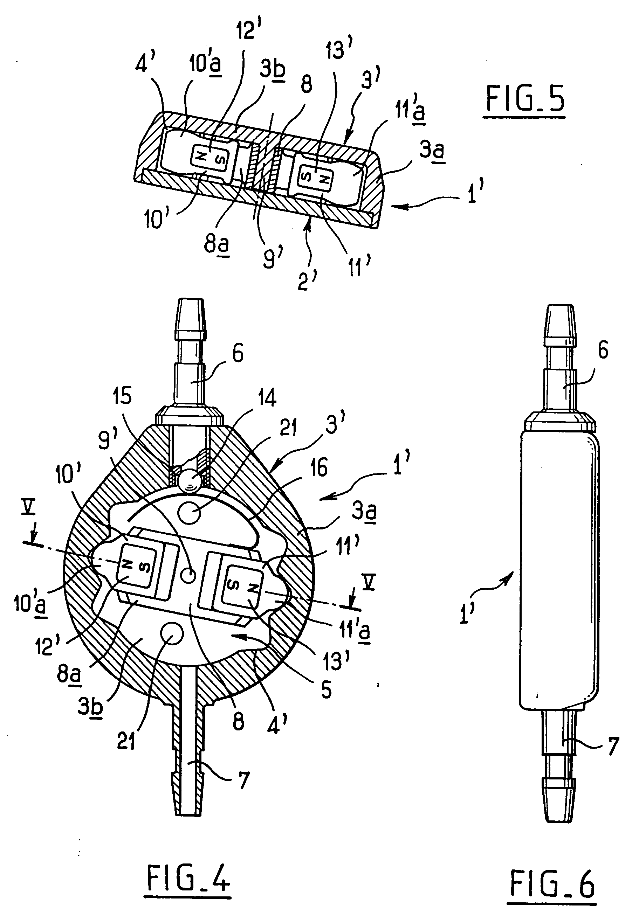

Sur les figures 4 à 6, on a représenté un second mode de réalisation du corps de valve 1' selon la présente invention.

Le corps de valve 1' est constitué d'un boîtier 3' et d'un couvercle supérieur 2' en forme de disque.

Le boîtier 3' comporte en saillie de la paroi inférieure 3b une partie centrale 9', formant l'axe central de la chambre 5.

Deux pièces 10' et 11' sont montées mobiles entre les branches latérales 8a du rotor 8, de part et d'autre de l'axe central 9'.

Les pièces mobiles 10' et 11' comportent respectivement un ergot 10'a et 11'a qui fait radialement saillie de la pièce mobile respective vers la paroi latérale 3a du boîtier 3' et logent chacune un micro-aimant 12' et 13', les micro-aimants étant disposés de manière à se repousser en étant de même polarité (S ou N).

Les moyens de verrouillage sont constitués par lesdits ergots 10'a et 11'a aptes à s'engager dans des cavités 4' ménagées sur la périphérie interne de la paroi latérale 3a de la chambre 5.

La chambre 5 comporte en outre des éléments de butée 21 en saillie de la paroi inférieure 3b du boîtier 3' pour limiter le déplacement en rotation du rotor 8.

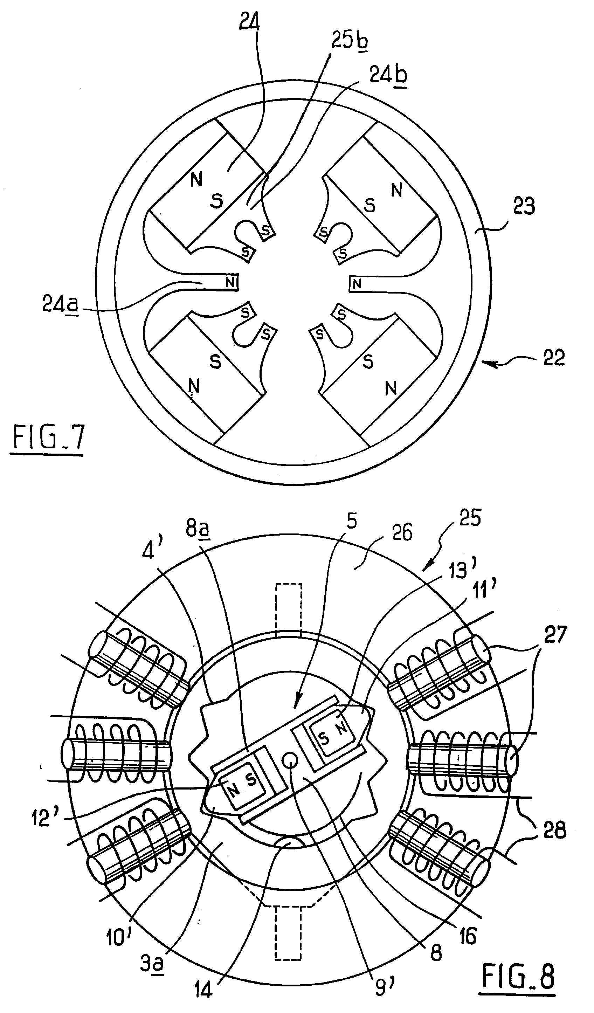

La figure 7 représente un dispositif de réglage externe 22 particulièrement adapté au second mode de réalisation de la valve selon la présente invention.

Le dispositif de réglage externe 22 est constitué d'un support annulaire 23 et d'aimants 24 dont les faces en regard sont de même polarité (N ou S), les lignes de champ de ces aimants sont canalisées par des pièces en fer doux 25a, 25b conçues de manière à réaliser un anneau magnétique formé d'une succession circulaire de pôles aimantés orientés radialement vers le centre du support 23. Ces pôles aimantés sont en outre disposés diamètralement deux par deux de telle manière que deux pôles situés sur un même diamètre soient de polarité identique et soient espacés d'une distance sensiblement égale ou supérieure à celle séparant les pôles extérieurs des deux micro-aimants mobiles 12' et 13' lorsque les ergots 10'a et 11'a sont engagés dans les cavités 4'.

On voit sur la figure 7, que deux pôles N sont entourés symétriquement de part et d'autre de quatre pôles S.

Sur la figure 8, on a représenté un troisième mode de réalisation d'un dispositif de réglage externe 25 selon la présente invention.

Le dispositif de réglage externe 25 comporte un support annulaire 26 et des moyens électromagnétiques montés circulairement sur ledit support 26.

Les moyens électromagnétiques sont constitués de barreaux magnétiques 27 autour desquels sont enroulés des bobinages 28.

Les barreaux 27 sont disposés radialement en direction des cavités de réception 4'.

On va maintenant décrire le fonctionnement de ces dispositifs de réglage externe.

La figure 1 représente le corps de valve 1 dans sa position verrouillée, les ergots 10a et 11a étant engagés dans les cavités 4.

Les micro-aimants verrouillent la valve suite à l'attraction magnétique engendrée par les polarités opposées des micro-aimants 12 et 13.

On détermine, d'une manière générale, la position du rotor dans la valve sous-cutanée au moyen d'une boussole appliquée à la verticale de la valve. Son repérage peut également être effectué par un système utilisant des sondes à effet Hall digitales associées à des diodes électroluminescentes ou par tout autre moyen électromagnétique.

La figure 3 représente le corps de la valve 1 dans sa position déverrouillée, les ergots 10a et 11a étant tous deux désengagés des cavités 4.

Pour déverrouiller la valve, on place le dispositif de réglage externe 17 illustré sur la figure 3 à la verticale du corps de valve 1 de sorte que les aimants 18 à 19 dont les faces en regard ont des polarités opposées (N et S) se positionnent symétriquement par rapport à l'axe central 9 de la chambre 5 et de part et d'autre des micro-aimants 12 et 13.

Compte-tenu de la différence de masse magnétique existant entre les aimants du dispositif de réglage externe et les micro-aimants de la valve, le simple fait d'approcher les pôles N et s du dispositif de réglage à proximité des pôles opposés S et N des micro-aimants, soumet les deux pôles extérieurs des micro-aimants à une attraction périphérique supérieure à l'attraction centrale des deux pôles intérieurs entre eux. Il en résulte un écartement symétrique et simultané des deux micro-aimants avec leurs pièces mobiles vers la périphérie de la chambre, ce qui déverrouille le rotor et le rend libre en rotation. Le rotor étant ainsi déverrouillé, il est possible de modifier sa position et par conséquent, la pression de fonctionnement et le débit de la valve, en faisant pivoter le dispositif de réglage autour de l'axe central de la chambre de la valve, ce qui entraîne le rotor simultanément. Pour verrouiller le rotor dans une nouvelle position déterminée, il suffit de retirer le dispositif de réglage en l'éloignant verticalement par rapport au plan de la valve. Les deux micro-aimants n'étant plus soumis à une attraction périphérique externe, se rapprochent alors l'un l'autre à nouveau sous l'effet de leur attraction mutuelle, provoquant ainsi le verrouillage des pièces mobiles.

Ce type de valve à micro-aimants mobiles ne peut pas être déréglé en présence d'un champ magnétique ou électromagnétique externe de forte puissance car les deux micro-aimants mobiles ne peuvent pas simultanément se désengager de leur cavité en présence d'un champ magnétique unidirectionnel.

Dans la mesure où les deux micro-aimants mobiles 12 et 13 sont disposés de part et d'autre de l'axe central de la valve, lorsque l'un des micro-aimants est attiré vers la périphérie de la chambre, l'autre est repoussé dans une cavité de verrouillage.

Il est nécessaire d'appliquer un dispositif de réglage particulier tel que celui illustré sur la figure 3 pour déverrouiller la valve, c'est-à-dire pour écarter les micro-aimants de façon symétrique.

Bien entendu, la puissance des aimants doit être supérieure à celle des micro-aimants de manière à vaincre la force d'attraction qui existe entre les micro-aimants.

La figure 4 représente le corps de valve 1' dans une position verrouillée, les ergots 10'a et 11'a étant engagés dans les cavités 4', du fait de la répulsion engendrée par la disposition des micro-aimants 12' et 13' dont les faces en regard sont de polarité identique (S).

Sur la figure 7, on a représenté un dispositif de réglage externe 22, apte à déverrouiller la valve 1' illustrée sur la figure 4.

Le déverrouillage s'effectue ici en plaçant le dispositif de réglage externe 22 à la verticale du corps de valve 1' de sorte que les deux pôles aimantés 25a de polarité identique (N) se positionnent symétriquement par rapport à l'axe 9' de la chambre 5 de part et d'autre des micro-aimants 12' et 13'.

Les micro-aimants 12' et 13' se désengagent alors des cavités 4' suite à la répulsion engendrée par la polarité identique existant entre les pôles aimantés 25a et les micro-aimants 12' et 13'.

Les micro-aimants 12' et 13' sont alors en équilibre instable et ont tendance à se déplacer par attraction vers le pôle aimanté 25b de polarité opposée (S) le plus proche, verrouillant ainsi le rotor dans une nouvelle position.

En faisant tourner le dispositif de réglage externe 22 autour de l'axe central 9', on provoque ainsi un déplacement du rotor 8 de cavité en cavité.

Tout comme le type de valve à verrouillage par attraction magnétique décrit précédemment en référence à la figure 1, ce type de valve à verrouillage par répulsion magnétique ne peut pas être non plus déréglé en présence d'un champ magnétique ou électromagnétique externe de forte puissance car les deux micro-aimants mobiles ne peuvent pas se désengager simultanément de leur cavité en présence d'un champ magnétique unidirectionnel. Dans la mesure où les deux micro-aimants mobiles 12' et 13' sont disposés de part et d'autre de l'axe central de la valve, lorsque l'un des micro-aimants est attiré vers le centre de la valve, l'autre est repoussé dans une cavité de verrouillage. Il est nécessaire d'appliquer un dispositif de réglage particulier tel que ceux illustrés sur la figure 7 et la figure 8 pour déverrouiller la valve, c'est-à-dire rapprocher les micro-aimants de façon symétrique. Bien entendu, le champ magnétique ou électromagnétique créé par le dispositif de réglage doit être plus puissant que celui existant entre les deux micro-aimants de manière à vaincre la force de répulsion qui les maintient écartés.

On a représenté sur la figure 8, un dispositif de réglage externe électromagnétique 25 superposé au second mode de réalisation de la valve selon l'invention, à savoir le corps de valve 1'.

Ce dispositif de réglage externe électromagnétique 25 n'est cependant pas limité au corps de valve 1' mais peut être aussi bien appliqué au premier mode de réalisation de la valve, à savoir le corps de valve 1.

Ce dispositif de réglage externe électromagnétique 25 peut être programmé à l'avance et il est apte à envoyer des séquences d'impulsions électromagnétiques codées correspondant aux différentes positions de verrouillage du rotor 8 dans la chambre 5 par l'intermédiaire de moyens électromagnétiques constitués par les barreaux magnétiques 27 et les bobinages 28.

Bien que l'invention ait été décrite en liaison avec des modes de réalisation particuliers, il est évident qu'elle n'y est nullement limitée et qu'on peut lui apporter de nombreuses variantes et modifications sans pour autant sortir de son cadre.

De même, la valve selon l'invention peut être utilisée dans des applications autres que le traitement de l'hydrocéphalie, et notamment par exemple pour la réalisation de sphincters urinaires artificiels ou de systèmes de distribution de médicaments, tels la morphine, l'insuline ou des médicaments anticancéreux.

## Revendications

1. Valve (1) sous-cutanée permettant le réglage externe d'un passage ou d'une distribution de liquide, ladite valve comportant un corps constitué d'une chambre interne (5) sensiblement cylindrique et plate, un conduit d'entrée (6) et un conduit d'évacuation (7) ménagés dans la paroi latérale (3a) de ladite chambre et aptes à être respectivement connectés à un cathéter d'amenée et un cathéter de drainage de liquide, un rotor apte à tourner dans ladite chambre autour de son axe central (9), un clapet anti-retour (14), tel une bille, formé au niveau de l'extrémité interne du conduit d'entrée, un ressort à lame courbe (16), de préférence semi-circulaire, fixé audit rotor, parallèle à la paroi latérale de la chambre et comprimant le clapet dans son siège de manière à réguler et le cas échéant bloquer le passage de liquide dans la chambre par le conduit d'entrée, deux micro-aimants (12, 13) montés dans le rotor et disposés de part et d'autre de l'axe central de la chambre et des moyens de verrouillage du rotor dans une position déterminée, caractérisée par le fait que les micro-aimants (12,13 ; 12', 13') sont mobiles linéairement dans ledit rotor (8) selon une direction radiale de celui-ci de manière à actionner lesdits moyens de verrouillage.

2. Valve selon la revendication 1, caractérisée par le fait que le rotor (8) est constitué d'un barreau en forme de H dont les branches latérales (8a) servent de moyens de guidage pour les micro-aimants mobiles (12,13 ; 12',13').

3. Valve selon l'une quelconque des revendications précédentes, caractérisée par le fait que les moyens de verrouillage comportent un ergot (10a,11a; 10'a,11'a) en saillie de pièces mobiles (10,11 ; 10',11') logeant chacune un micro-aimant (12,13 ; 12',13') et des cavités (4,4') de réception des ergots ménagées circulairement dans la chambre (5), lesdites cavités (4 ; 4') ayant une forme adaptée pour retenir lesdits ergots (10a,11a; 10'a,11'a).

4. Valve selon la revendication 3, caractérisée par le fait que les micro-aimants mobiles (12,13) sont disposés de manière à s'attirer, leurs faces en regard étant de polarités opposées, et que les cavités (4) sont ménagées sur la périphérie d'une partie centrale (2a) de la paroi supérieure (2) ou inférieure (3b) du corps de valve (1), les ergots (10a,11a) faisant saillie des pièces mobiles (10,11) respectives perpendiculairement à ladite paroi supérieure (2) ou inférieure (3b).

5. Valve selon la revendication 4, caractérisée par le fait que la paroi supérieure (2) ou inférieure (3b) du corps de valve (1) est constituée d'un couvercle amovible qui comporte ladite partie centrale (2a).

6. Valve selon la revendication 3, caractérisée par le fait que les micro-aimants mobiles (12',13') sont disposés de manière à se repousser, leurs faces en regard étant de même polarité, et que lesdites cavités (4') sont ménagées sur la périphérie de la paroi latérale (3a) du corps de valve (1'), les ergots (10'a, 11'a) faisant radialement saillie des pièces mobiles (10',11') respectives vers ladite paroi latérale (3a).

7. Dispositif de réglage externe (17) de la valve selon la revendication 4 ou 5 ayant des aimants de masse magnétique supérieure à celle des micro-aimants mobiles (12,13), lesdits aimants (18,19) étant montés sur un support commun (20), de préférence annulaire ou en forme de fer à cheval, caractérisé par le fait que le dispositif est constitué de deux aimants (18,19) dont les faces frontales en regard sont de polarités opposées et espacés d'une distance sensiblement égale ou supérieure à la distance séparant les pôles extérieurs des deux micro-aimants mobiles (12,13) lorsque ces derniers actionnent les moyens de verrouillage.

8. Dispositif de réglage externe (22) de la valve selon la revendication 6, caractérisé par le fait qu'il est constitué d'une succession circulaire de pôles aimantés (24a,24b), montés sur un support (23), de préférence annulaire, les pôles de même polarité étant disposés diamétralement deux par deux et espacés d'une distance sensiblement égale ou supérieure à celle séparant les pôles extérieurs des deux micro-aimants mobiles (12',13') lorsque ces derniers actionnent les moyens de verrouillage.

9. Dispositif de réglage externe (25) de la valve selon l'une quelconque des revendications 3 à 6, caractérisé par le fait qu'il comporte un support (26), de préférence annulaire, et des moyens électromagnétiques (27,28) montés circulairement sur ledit support (26), et disposés radialement en direction des cavités (4;4') de réception des ergots (10a,11a;10'a,11'a), lesdits moyens électromagnétiques (27,28) étant aptes à envoyer des séquences d'impulsions électromagnétiques codées correspondant aux différentes positions de verrouillage du rotor (8) dans la chambre (5).

## Claims

1. Subcutaneous valve (1) permitting external control of a flow or supply of liquid, said valve comprising a body formed by a substantially cylindrical and flat internal chamber (5), an inlet duct (6) and an outlet duct (7) formed in the side wall (3a) of said chamber and designed to be connected respectively to a supply catheter and a drainage catheter for liquid, a rotor designed to turn in said chamber about its central axis (9), a non-return valve (14), such as a ball, formed level with the internal end of the inlet duct, a curved leaf spring (16), preferably semi-circular, fixed to said rotor, parallel to the side wall of the chamber and compressing the valve in its seat so as to regulate and if necessary block the flow of liquid into the chamber through the inlet duct, two micromagnets (12, 13) fitted in the rotor and disposed on either side of the central axis of the chamber and means for locking the rotor in a given position, characterised by the fact that the micromagnets (12, 13; 12', 13') are mobile linearly in said rotor (8) in a radial direction of the latter so as to operate said locking means.

2. Valve according to claim 1, characterised by the fact that the rotor (8) is formed by a H-shaped bar the side members (8a) of which serve as guiding means for the mobile micromagnets (12, 13; 12', 13').

3. Valve according to any one of the preceding claims, characterised by the fact that the locking means comprise a lug (10a, 11a; 10'a, 11'a) projecting from mobile parts (10, 11; 10', 11') each housing a micromagnet (12, 13; 12', 13') and cavities (4, 4') for reception of the lugs formed circularly in the chamber (5), said cavities (4; 4') having a shape adapted to retain said lugs (10a, 11a; 10'a, 11'a).

4. Valve according to claim 3, characterised by the fact that the mobile micromagnets (12, 13) are disposed so as to attract one another, their opposing faces being of opposite polarity, and that the cavities (4) are formed on the periphery of a central portion (2a) of the upper wall (2) or lower wall (3b) of the valve body (1), the lugs (10a, 11a) projecting from the respective mobile parts (10, 11) perpendicularly to said upper wall (2) or lower wall (3b).

5. Valve according to claim 4, characterised by the fact that the upper wall (2) or lower wall (3b) of the valve body (1) is constituted by a detachable cover which comprises said central portion (2a).

6. Valve according to claim 3, characterised by the mobile micromagnets (12', 13') are disposed so as to repel one another, their opposing faces being of the same polarity, and that said cavities (4') are formed on the periphery of the side wall (3a) of the valve body (1'), the lugs (10'a, 11'a) projecting radially from the respective mobile parts (10', 11') towards said side wall (3a).

7. Device for external control (17) of the valve according to claim 4 or 5 having magnets of magnetic mass greater than that of the mobile micromagnets (12, 13), said magnets (18, 19) being mounted on a common support (20), preferably annular or in the shape of a horseshoe, characterised by the fact that the device is constituted by two magnets (18, 19) the opposing front faces of which are of opposite polarity and spaced by a distance substantially equal to or greater than the distance separating the outer poles of the two mobile micromagnets (12, 13) when the latter operate the locking means.

8. Device for external control (22) of the valve according to claim 6, characterised by the fact that it is constituted by a circular succession of magnetised poles (24a, 24b) mounted on a support (23), preferably annular, the poles of the same polarity being disposed diametrically two by two and spaced by a distance substantially equal to or greater than that separating the outer poles of two mobile micromagnets (12', 13') when the latter operate the locking means.

9. Device for external control (25) of the valve according to any one of claims 3 to 6, characterised by the fact that it comprises a support (26), preferably annular, and electromagnetic means (27, 28) mounted circularly on said support (26) and disposed radially in the direction of the cavities (4; 4') for reception of the lugs (10a, 11a; 10'a, 11'a), said electromagnetic means (27, 28) being designed to send sequences of coded electromagnetic pulses corresponding to the different locking positions of the rotor (8) in the chamber (5).

## Patentansprüche

1. Subkutanes Ventil, das die externe Einstellung eines Durchflusses oder einer Verteilung einer Flüssigkeit gestattet, welches Ventil aufweist: einen Körper, der durch eine im wesentlichen zylindrische und flache innere Kammer (5) gebildet wird, eine Einlaßleitung (6) und eine Auslaßleitung (7), die in der Seitenwand (3a) dieser Kammer ausgebildet und dazu eingerichtet sind, an einen Zufuhrkatheter bzw. einen Rücklaufkatheter für Flüssigkeit angeschlossen zu werden, einen Rotor, der in der Kammer um seine Mittelachse (9) drehbar ist, ein am inneren Ende der Einlaßleitung gebildetes Rückschlagventil (14), etwa ein Kugelventil, eine gekrümmte, vorzugsweise halbkreisförmige Blattfeder (16), die an dem Rotor befestigt ist, parallel zur Seitenwand der Kammer verläuft und das Rückschlagventil gegen seinen Sitz vorspannt, um den Durchfluß der Flüssigkeit aus der Einlaßleitung in die Kammer zu steuern und gegebenenfalls zu sperren, zwei Mikromagnete (12, 13), die in dem Rotor montiert und beiderseits der Mittelachse der Kammer angeordnet sind, und Mittel zur Verriegelung des Rotors in einer bestimmten Position, dadurch gekennzeichnet, daß die Mikromagnete (12, 13; 12', 13') in dem Rotor (8) in einer radialen Richtung desselben linear beweglich sind, um die genannten Verriegelungsmittel zu betätigen.

2. Ventil nach Anspruch 1, dadurch gekennzeichnet, daß der Rotor (8) durch einen H-förmigen Stab gebildet wird, dessen seitliche Arme (8a) als Mittel zur Führung der beweglichen Mikromagnete (12, 13; 12', 13') dienen.

3. Ventil nach einem der vorstehenden Ansprüche, dadurch gekennzeichnet, daß die Verriegelungsmittel einen Nocken (10a, 11a; 10'a, 11'a), der von beweglichen Teilen (10, 11; 10', 11') vorspringt, die jeweils einen Mikromagneten (12, 13; 12' 13') aufnehmen, und Höhlungen (4, 4') zur Aufnahme der Nocken aufweist, die kreisförmig in der Kammer (5) ausgebildet sind, welche Höhlungen (4, 4') eine Form haben, die dazu angepaßt ist, die genannten Nocken (10a, 11a; 10'a, 11'a) zu halten.

4. Ventil nach Anspruch 3, dadurch gekennzeichnet, daß die beweglichen Mikromagnete (12, 13) so angeordnet sind, daß sie sich anziehen, wobei ihre gegenüberliegenden Flächen entgegengesetzte Polarität haben, und daß die Höhlungen (4) im Umfang eines zentralen Bereichs (2a) der oberen Wand (2) oder der unteren Wand (3b) des Körpers (1) des Ventils ausgebildet sind, wobei die Nocken (10a, 11a) rechtwinklig zu der genannten oberen Wand (2) oder unteren Wand (3b) von den jeweiligen beweglichen Teilen (10, 11) vorspringen.

5. Ventil nach Anspruch 4, dadurch gekennzeichnet, daß die oberen Wand (2) oder die untere Wand (3b) des Körpers des Ventils (1) durch einen Deckel gebildet wird, der den genannten zentralen Bereich (2a) aufweist.

6. Ventil nach Anspruch 3, dadurch gekennzeichnet, daß die beweglichen Mikromagnete (12', 13') so angeordnet sind, daß sie sich abstoßen, wobei ihre gegenüberliegenden Flächen dieselbe Polarität haben, und daß die genannten Hohlräume (4') im Umfang der Seitenwand (3a) des Körpers des Ventils (1') ausgebildet sind, wobei die Nocken (10'a, 11'a) radial in Richtung auf die genannte Seitenwand (3a) von den jeweiligen beweglichen Teilen (10', 11') vorspringen.

7. Vorrichtung (17) zur externen Einstellung des Ventils nach Anspruch 4 oder 5, mit Magneten von größerer magnetischer Masse als die beweglichen Mikromagnete (12, 13), welche Magnete (18, 19) an einem gemeinsamen, vorzugsweise kreisförmigen oder hufeisenförmigen Träger (20) montiert sind, dadurch gekennzeichnet, daß die Vorrichtung durch zwei Magnete (18, 19) gebildet wird, deren gegenüberliegende Stirnflächen entgegengesetzte Polarität haben und einen Abstand zueinander aufweisen, der im wesentlichen gleich oder größer ist als der Abstand zwischen den äußeren Polen der beiden beweglichen Mikromagnete (12, 13), wenn diese letzteren die Verriegelungsmittel betätigen.

8. Vorrichtung (22) zur externen Steuerung des Ventils nach Anspruch 6, dadurch gekennzeichnet, daß sie durch eine kreisförmige Folge von Magnetpolen (24a, 24b) gebildet wird, die an einem vorzugsweise kreisförmigen Träger (23) montiert sind, wobei die Pole von gleicher Polarität paarweise diametral angeordnet sind und einen Abstand zueinander aufweisen, der im wesentlichen gleich oder größer ist als der Abstand zwischen den äußeren Polen der beiden beweglichen Mikromagnete (12', 13'), wenn diese letzteren die Verriegelungsmittel betätigen.

9. Vorrichtung (25) zur externen Steuerung des Ventils nach einem der Ansprüche 3 bis 6, dadurch gekennzeichnet, daß sie einen vorzugsweise kreisförmigen Träger (26) und elektromagnetische Mittel (27, 28) aufweist, die kreisförmig an dem Träger (26) montiert und radial in Richtung der Höhlungen (4, 4') zur Aufnahme der Nocken (10a, 11a; 10'a, 11'a) angeordnet sind, wobei die elektromagnetischen Mittel (27, 28) dazu ausgebildet sind, elektromagnetische Impulsfolgen abzugeben, die entsprechend den unterschiedlichen Verriegelungspositionen des Rotors (8) in der Kammer (5) kodiert sind.
